# EUROPEAN PATENT APPLICATION

(11) **EP 4 691 432 A2**
(43) Date of publication of application: **11.02.2026**
(21) Application number: 25194421.1
(22) Date of filing: 06.08.2025
(51) Int. Cl.: A61F 5/56

(54) **SNORING AND OBSTRUCTIVE SLEEP APNEA PREVENTION DEVICE**

(30) Priority: 06.08.2024 US 202418795333
(71) Applicant: Naidu, Prakash, Crj, Ottawa, Ontario K2C 0J8 (CA); Liu, Xingchen, Melbourne, 3000 (AU); Haats, Tim, Ottawa K2G 2T5 (CA); Karaceper, Caner, Ottawa, Ontario K1H 7Y7 (CA); Karaceper, Cem, Ottawa, Ontario K1H 7X1 (CA); Dzafic, Anida, Ottawa, Ontario K1H 7Y7 (CA)
(72) Inventor: Naidu, Prakash, Crj, Ottawa, Ontario K2C 0J8 (CA); Liu, Xingchen, Melbourne, 3000 (AU); Haats, Tim, Ottawa K2G 2T5 (CA); Karaceper, Caner, Ottawa, Ontario K1H 7Y7 (CA); Karaceper, Cem, Ottawa, Ontario K1H 7X1 (CA); Dzafic, Anida, Ottawa, Ontario K1H 7Y7 (CA)
(74) Representative: Hofstetter, Schurack & Partner

(57) **Abstract**

An oral appliance (OA) made as upper and lower splints or denture set of a user to facilitate adjustable mandibular advancement using an adjustable strap system assisting in partially or fully reducing snoring and thus ameliorating obstructive sleep apnea (OSA). The mandibular advancement is realized by a single adjustable strap avoiding providing a set of straps. Excess recess in strap assembly facilitates variation in mandibular advancement even after locking gross length of strap. An optional additional modular breathing assist device is disclosed that may serve the purpose of controlling quantity of air flow from mouth or oral cavity of a user during exhaling from the airway enhancing the anti-snoring function.

## Description

### FIELD OF THE INVENTION

The present invention pertains to oral appliances, and in particular to oral appliances for the prevention of snoring, sleep apnea, or similar other conditions.

### BACKGROUND OF THE INVENTION

It is well known and documented that those individuals suffering from sleep-related breathing disorders such as sleep apnea exhibit moderate to excessive snoring which is often ignored or neglected without any preventive or curative measures. It is also well known that this neglect is causing an increased risk resulting in more severe health problems later on such as stroke, atrial fibrillation, hypertension, chronic heart failure, excessive sleepiness in daytime causing driving accidents or equipment operation accidents, and a variety of ailments e.g. diabetes, depression, among others including the fact that an obstructive sleep apnea (OSA) episode can be life threatening; however, 80 percent of the cases of moderate and severe obstructive sleep apnea is likely undiagnosed (Source: American Sleep Apnea Association: Sleep Apnea Information for Clinicians- A very short course on sleep apnea; Hu, Pub. No. US 2020/0129278 dated Apr. 30, 2020). Studies have shown, for instance, that individuals demonstrating snoring are most likely to be suffering from OSA that may lead to one or more of the aforementioned health problems. OSA is characterized by periodic and repetitive collapse of the upper airway during sleep, the frequency of the same is specified as apnea and hypopnea index (AHI) that shows the average number of apneas and hypopnea per hour of sleep. Health care professionals use the terms of respiratory disturbance index (RDI), and AHl interchangeably. While apnea episodes entail total stoppage or pause in breathing typically for 20 to 40 seconds, hypopnea episodes entail typically shallow or partial breathing accompanying 30% or greater reduction in air flow lasting for 10 seconds or longer with an associated greater desaturation in the person's oxygen levels exceeding 4% often resulting in fragmentation of sleep or arousal. The AHI is calculated by dividing the sum of apneas and hypopneas by the number of hours of sleep. AHI values are categorized as follows: severe if more than 30 episodes per hour, moderate if episodes ranging from 15 to 30 per hour, and mild if 5 to 15 episodes per hour. These episodes of reduced airflow are typically caused by the relaxation of the tongue and pharyngeal walls to varying degrees during the several stages of sleep wherein the musculature supporting these tissues relaxes, therefore, as air is inhaled, the tongue and posterior walls of the pharynx collapse, causing snoring that is in fact vibration noise caused by partial or complete obstruction of the airway. The episodes may cause several undesirable conditions such as reduced oxygen level in the blood (hypoxemia), elevated circulating carbon dioxide (hypercapnia), and arousal from sleep to re-establish a stable airway.

A rigorous study published in 2019 in Lancet reports (Markets and Markets. Sleep Apnea Devices Market, Report Code: MD 1181, Published Date: Aug 2021) that nearly 1 billion adults aged 30-69 years worldwide could have OSA, and the number of people with moderate to severe OSA, for which treatment is generally recommended, is estimated to be almost 425 million. Major part of the actual market remains untapped or not served as a large number of patients remain undiagnosed (Adam V Benjafield, Najib T Ayas, Peter R Eastwood, Raphael Heinzer, Mary S M Ip, Mary J Morrell, Carlos M Nunez, Sanjay R Patel, Thomas Penzel, Jean-Louis D Pépin, Paul E Peppard, Sanjeev Sinha, Sergio Tufik, Kate Valentine, and Atul Malhotra. Estimation of the global prevalence and burden of obstructive sleep apnoea: a literature-based analysis. Lancet Respir Med. 2019 Aug; 7(8): 687-698) as they do not even know the condition and risks because the symptoms arise only while the person is in sleep and often does not believe when very limited close individuals, such as a bed partner may dare to point out, more due to noise creation annoyance such as snoring or in some cases combined with grinding of teeth (known as bruxism) and seldom due to genuine health concerns of the sufferer. The highest number of sufferers are in China, USA, Brazil, and India in decreasing order, with USA estimated to have more than 54.1 million with AHI of 5 or more events per hour; and more than 23.6 million with AHI of 15 or more events per hour. Some estimates are even higher, for example, Ramabadran reported in US Patent No. 11,147,506 dated Oct. 19, 2021 titled "Sleep diagnostic system and method" that close to 65 million American adults suffer from sleep disordered breathing conditions where 1 in 5 adults has obstructive sleep apnea or central sleep apnea conditions and contemplated that out of this large number of estimated sufferers, probably just 1 in 15 adults are properly screened, tested and then treated for this condition, leaving a substantially huge untested and thus unattended population. Canada has a substantial number of affected persons: more than 4.7 million with AHI of 5 or more events per hour; and more than 0.9 million with AHI of 15 or more events per hour. The extent and nature of the problem has attracted ongoing discussions on need for change in the way in which the diagnosis and treatment is currently funded by public health and private insurance coverage (Sachin R. Pendharkar, Marcus Povitz, Nick Bansback, Charles F.P. George, Debra Morrison, Najib T. Ayas for the Canadian Sleep and Circadian Network. Testing and treatment for obstructive sleep apnea in Canada: funding models must change. CMAJ 2017 December 11;189: E1524-8. doi: 10.1503/cmaj.170393).

While severe sleep-related breathing disorders may require Continuous Positive Airway Pressure (CPAP) therapy, less severe sleep-related breathing disorders may be treated with other therapies or devices, such as an Oral Appliance (OA). Generally, an oral appliance includes either a single mono-block device to impart forward advancement of the user's lower jaw (mandible) relative to the user's upper jaw (maxilla); or alternatively a duo-bloc device wherein upper and lower dental trays are coupled together in such a way so as to achieve similar mandibular advancement. Accordingly, an OA is generally referred to as a Mandibular Advancement Device (MAD) or a Mandibular Advancement Splint (MAS). Unblocking of a user's airway during sleep is achieved by the forward advancement of the mandible that helps prevent the soft tissue of the tongue and the throat from collapsing into the airway passage. In an OA, it is important to achieve the correct amount of forward mandibular advancement. For example, the soft tissue of a user's tongue and throat may not be prevented from collapsing into the user's airway and thus they may continue to Suffer from a sleep-related breathing disorder if the forward mandibular advancement imparted by the OA is inadequate. Conversely, a user may experience undesirable discomfort in the mandibular musculature, tooth pain, or even more serious side effects such as temporomandibular joint (TMJ) pain if the forward mandibular advancement imparted by the OA is excessive (as reported, for example, by Marklund, M.; Franklin, K.A. Long-term effects of mandibular repositioning appliances on symptoms of sleep apnoea. J. Sleep Res. 2007, 16, 414-420; and Pliska, B.T.; Nam, H.; Chen, H.; Lowe, A.A.; Almeida, F.R. Obstructive sleep apnea and mandibular advancement splint: Occlusal effects and progression of changes associated with a decade of treatment. J. Clin. Sleep Med. 2014, 10, 1285-1291). In extreme cases, the discomfort may cause the user to stop wearing the OA all together, in many other cases, it is reported that undesirable arousal from sleep may occur.

Some oral appliances have been disclosed that include mechanisms for adjusting the amount of mandibular advancement provided by the oral appliance. These are called adjustable or titratable devices.

Halstrom describes in U.S. Patent No. 5,365,945 dated Nov. 22, 1994 titled "Adjustable Dental Appliance for Treatment of Snoring and Obstructive Sleep Apnea" an appliance that consists of an upper bite block conforming to the patient's maxillary dentition, a lower bite block conforming to the patient's mandibular dentition, and a connecting assembly secured to an anterior region of the upper and lower bite blocks for adjustably or in a titratable manner coupling the upper and lower bite blocks together.

Lowe describes in WO1995019746 dated 27 July 1995 titled "Mandible repositioning appliance for snoring prevention" a device formed by an upper bite block and a lower bite block interconnected by an extendable connector including a posterior section connected to the rear portion of the upper bite block and an anterior section connected to the front portion of the lower bite block and an adjustable threaded element interconnection between the anterior and posterior sections affecting the relative positions.

Fenton describes in U.S. Patent No. 5,499,633 dated Mar. 19, 1996 titled "Anti-snoring Device with Adjustable Upper and Lower Relational Members" an anti-snoring device that comprises an upper member for receiving upper teeth of the user, which is joined in a spaced relationship to a lower member for receiving lower teeth of the user. The lower member is selectively positioned with respect to the upper member such that the lower member, by receiving the lower teeth of the user, moves the lower jaw of the user to a position that is forward of its "normal' resting or biting position. This forward movement of the lower jaw is claimed to significantly open the airway of the user, thereby alleviating, reducing, or eliminating snoring.

Kidd and Lane describe in U.S. Patent No. 5,829,441 dated Nov. 3, 1998 titled "Customizable Dental Device for Snoring and Sleep Apnea Treatment" a mandible extension dental device which includes adjustable upper and lower arch trays and coupling means designed to pull the lower jaw of a user forwardly during Sleep, so as to minimize snoring and mild apnea conditions. The device is intended to be rapidly fitted in the field without the need for the services of a dentist.

Halstrom describes in U.S. Patent No. 5,868,138 dated Feb. 9, 1999 titled "Dental Appliance for Treatment of Snoring and Obstructive Sleep Apnea" yet another configuration of a dentally retained intra-oral appliance worn at night for treatment of snoring and obstructive sleep apnea.

Strong describes in U.S. Patent No. 6,526,982 dated Mar. 4, 2003 titled "Anti-snoring Device and Method of Making Same" an anti-snoring device that has maxillary and mandibular bite forms with outwardly extending pivots which are mounted to the bite forms by frameworks which are at least partially embedded in the bite forms supposedly able to retain the pivots securely in place even in the face of extensive bruxing.

Palmisano describes in U.S. Patent No. 6,536,439 dated Mar. 25, 2003 titled "Apparatus and methods for treatment of conditions including obstructive sleep apnea and snoring" a maxillary expansion device that is fitted to teeth of the upper jaw, and the maxilla is expanded by operation of a jack screw, such that, after the maxilla stabilizes, the minimum cross-sectional area of the nasal cavity increases, reducing nasal airway resistance resulting in curing, or ameliorating, obstructive sleep apnea and/or snoring.

Palmisano describes in U.S. Patent No. 6,604,527 dated Aug. 12, 2003 titled "Mandibular Advancement Device" a custom made device made for each user from a desired cast of the user's jaw having a lower bite block, with a pair of upwardly extending flanges, and an upper bite block, with a pair of downwardly extending flanges; the flanges each carry engagement surfaces. When the bite blocks are fitted to the jaws of a user during sleep, the lower and upper engagement surfaces engage and cause anterior advancement of the lower jaw from the reflex path of opening and maintain that engagement and advancement whilst permitting movement, up to the normal range of jaw opening.

Palmisano and Mehta describe a version of the above in EP 1094761 published dated 29 Oct. 2008. Bedford discloses some updates and improvements over Palmisano described above, in US 2018/0360646 dated Dec. 20, 2018 and in AU 2021250941 dated Oct. 15, 2021 titled "A Mandibular Advancement Device". Introduction of a ratchet mechanism to effect the mandibular advancement along with the lower and upper parts being formed from relatively hard lower and upper shells, relatively soft lower and upper linings, and heat formable lower and upper inner linings therebetween appear to be the newly claimed innovative steps.

Scarberry et.al. describe in U.S. Patent No. 8,205,617 dated June 26, 2012 titled "Oral Appliance for Treatment of Snoring and Sleep Apnea" an oral appliance system that comprises an upper tray and a plurality of lower trays. The upper tray is adaptable to conform to a user's maxillary dentition and each of the lower trays is adaptable to conform to the user's mandibular dentition. The lower trays are structured to engage the upper tray and each of the lower trays is structured to impart a different fixed amount of mandibular advancement and/or different amount of vertical height.

Kopp describes in U.S. Patent No. 8,640,705 dated Feb. 4, 2014 titled "Anti-Snoring Device" an arrangement for mandibular advancement wherein elongated holes and so called S-shaped design of a pair of protrusion ties in conjunction with U-shaped upper and lower jaw bars are claimed to provide elasticity and thus spring action.

Fallon, Jung, and Fallon describe in U.S. Patent No. 8,833,374 dated Sep. 16, 2014 titled "Intra-oral Mandibular Advancement Appliance" a mandibular advancement device wherein upper and lower tray assemblies having shapes against which the teeth of user's upper and lower jaws are seated respectively may be adjusted and locked in different positions respective with each other. A tongue rest is also provided to prevent tongue from falling across and blocking the patient's airway.

Petelle and Fleury describe in U.S. Patent No. 9,545,330 dated Jan. 17, 2017 titled "Intraoral orthosis, a method of fabricating such an orthosis, and a method of adjusting it" an intraoral orthosis for treating snoring and sleep-apnea syndrome that includes two shells connected together by an adjustable connection device, the adjustable connection device including two notched tabs that are arranged respectively on either side of one of the shells, each notched tab co-operating in adjustable manner with a respective adjustment housing, arranged correspondingly on either side of the other of the shells, the adjustment housings being molded integrally with the other shell, each adjustment housing being connected to the other shell by a foldable support structure. A version of this was earlier described in U.S. Patent No. 9,095,454 dated Aug. 4, 2015 titled the same.

Kim et. al. describe in U.S. Patent No. 9,949,868 dated Apr. 24, 2018 titled "Mandibular Advancement Device" a configuration wherein relative positions of upper and lower fins on upper and lower splints enable desired mandibular advancement, claiming manufacturing a set of at least two devices following a process for digital design based on obtaining electronic data on the shape of user's dentition as the invention.

Baratier and Palomino describe in U.S. Patent No. 10,363,160 dated Jul. 30, 2019 titled "Mandibular Repositioning Device" an intra-oral device comprising an upper splint structured to engage with one or more teeth on user's maxilla, a lower splint structured to engage with one or more teeth on user's mandible, a pair of lateral connecting rods (each connecting rod has a first rod end that connects to the lower splint and a second rod end that connects to the upper splint), and the connecting rods are configured to maintain the mandible in an advanced position relative to the maxilla.

Veis and Ataii describe in U.S. Patent No. 10,363,161 dated Jul. 30, 2019 titled "Sleep Apnea Oral Appliance for Use During Orthodontic Treatment" a sleep apnea oral appliance in which a pair of appliance trays are worn over orthodontic trays coming in contact with maxillary dentition and mandibular dentition respectively that may comprise a series of different configurations in order to change the position of a user's teeth and/or the shape of the user's jaw.

Fallon describes in U.S. Patent No. 10,849,783 dated Dec. 1, 2020 titled "Full Movement Jaw Advancement Oral Appliance to Reduce the Effects of Snoring and/or Sleep Apnea" an oral appliance that includes an upper arch tray assembly against which the user's upper teeth carried are received, a lower arch tray assembly against which the user's lower teeth are received, and an intermediate guide post support tray that is connected at the bottom thereof to the lower arch tray assembly and at the top thereof to the upper arch tray assembly by an upper arch tray retaining post that extends therebetween.

Mortadi describes in U.S. Patent 10,888,451 dated Jan.12, 2021 titled "Oral Device for Preventing Sleep Apnea and a Method of Manufacturing" an oral device that provides an upper plate and lower plate, each adapted to retain the biting surface of the respective set of maxilla and mandible teeth, wherein a pair of S-shaped curved rods pivotally interconnecting both plates are adapted for maintaining an operable spaced apart relationship therebetween.

Radmand describes in U.S. Patent 11,191,663 dated Dec. 7, 2021 titled "Oral Appliance and Kit for Treatment of Sleep Apnea" an oral appliance for the treatment of obstructive sleep apnea that includes a mouthpiece embedded with a variety of sensors and a stimulator configured for being received in an oral cavity of a user.

Kopelman describes in U.S. Patent No. 11,207,208 dated Dec. 28, 2021 titled "Systems and Methods for Positioning a Patient's Mandible in Response to Sleep Apnea Status" three different optional embodiments wherein a rotor element, or a linear advancement structure, or a tether element winding and unwinding are deployed along with use of actuator to achieve relative advancement of the bottom part against the upper part.

Hart et. al. describe some configurations of airflow control separately or integrated with MAD in US Patent No.10, 010,444 dated Jul. 3, 2018 titled "Breathing Assist Device", US 2018/0207020 published dated Jul. 26, 2018 titled "Breathing Assistance Apparatus", and other patents/ applications related to airflow control valve arrangements, e.g. AU2019235611, CA3093591, CN201980018923.0, EP1976751, JP2020-545633, KR10-2020-7028700, NZ767473, US16/980,142. However, they are not modular enabling attachment to any other Oral Appliance, need to use a set of valves that require to be changed, thus inconvenient to use.

These and similar other oral appliances, however, are all directed for users with natural teeth as the devices are meant to be worn over the mandibular dentition and maxillary dentition. Moreover, they are expensive, complicated to manufacture, and have adjustment mechanisms that are difficult to operate. Furthermore, the OAs currently available that incorporate adjustment mechanisms tend to be bulky, thereby adversely impacting the user's comfort level, additionally, these known OAs are difficult to properly fit, do not allow sufficient vertical or horizontal movement of the jaw when worn, and are not flexible enough to allow to be suitable for those who generally wear standard ordinary, or implant retained dentures also known as overdentures in the art.

In particular, the adjustment mechanisms in many of the disclosures involves provision for a set of straps, for example a set of six to nine straps, from which an appropriate strap is chosen by the user or the attending care giver such as a dentist or denturist depending on the appropriate length of strap established during trials for fulfilling the required mandibular advancement. This is inconvenient due to the need for replacement of a strap by the user. It is also contributing to unnecessary waste as the unutilized straps are adding up to the garbage thus impacting the environment adversely.

It would also be desirable to have an additional optional attachment means to control the exhaled breathing out air quantity that can provide resistance to the air flow and help open the airway as a complementary effort to the opening of airway by mandibular advancement. Known in the art as EPAP (Expiratory Positive Airway Pressure) therapy, this approach is emerging as an effective treatment for snoring and obstructive sleep apnea, and is considered as an alternative to CPAP at least for mild to moderate cases, but currently there is no convenient accessory that can be attached to any oral appliance as an add-on attachment with ease of control of quantity of air flow.

Accordingly, a need exists for adjustable means for an oral appliance and accessories enabling addition of EPAP therapy for the avoidance of snoring and thus possible preventive measures or treatment of OSA that can provide a solution for user market segments while overcoming at least partially the aforementioned limitations and other shortcomings associated with known oral appliances.

Wearable oral device solution OAs for avoiding snoring and thus possibly OSA such as Mandibular Advancement Devices (MADs) are typically very similar to dentures and can be suggested by a dentist or even a denturist for use avoiding the logistical complexity of sleeping overnight in a lab for the Level 1 test required for a physician prescribed intervention. Broad market under sleep disorders includes diagnosis at different levels of complexity (Level 1, Level 2, Level 3 and Level 4 in decreasing order of complexity) interventions followed by CPAP as a directly physician recommended primary option and subsequently moving on to an OA for mainly those who do not tolerate or do not prefer CPAP. Considerable number of users of CPAP machines have expressed that there are many problems with using them, for example, difficulty getting the right style and size of mask, difficulty in getting used to wearing the device during sleep, difficulty tolerating forced air, dry nose and/or mouth, feeling claustrophobic, mask leak, skin irritation or pressure sores, difficulty falling asleep, and many such inhibitions. The difficulties inherent to CPAP machines lead to a high failure rate perhaps as much as 50% among patients using them (Hu, Pub. No. US 2020/0129278 dated Apr. 30, 2020). This current approach of CPAP as first line of OSA treatment that too only after the situation becomes serious appears to be the main cause for the massive number of undiagnosed sufferers (Benjafield et. al., Lancet, 2019).

Senior citizens (considered as 60+ years in some countries, but may vary from country to country such as lower or higher such as 65+ years in some countries) and near senior citizens (for example 50+ to 55+ years age up to senior citizen age) constitute a large demographic group that may have a need for full or partial dentures. According to several estimates quoting the Journal of Prosthetic Dentistry, 37.9 million Americans were estimated to require dentures in 2020 (Douglass et.al. "Will there be a need for complete dentures in the United States in 2020?", J. Prosthet Dent. 2002 Jan;87(1):5-8. doi: 10.1067/mpr.2002.121203). That's nearly 9% of the United States population. As Canadian population is approximately 0.11 of the USA population (Simon Fraser University. Population Comparison of Canada, United States, and Mexico: Profile Report, World Population), assuming similar North American demographics, the estimated denture users in Canada are 4.17 million, adding up to about 42 million across North America.

There is a significant need of the denture wearing market for devices to address the annoyance of snoring noise creation, while also safeguarding one from OSA, whether or not diagnosed. It may be noted that all currently available MADs in market described in the earlier section of this disclosure are worn over the natural teeth, and thus there is a need for a product that can be worn by the denture wearing market segment.

The only option that comes closest to the needs of this market segment that is available currently for full denture wearers is from an Australia headquartered company offered as Somnomed^{®} Edentulous^{™} costing a considerable amount. However, this product is not part of a denture or cannot be worn over denture; it is an additional product that needs the user to remove their regular denture and to obtain and wear this expensive device separately. Another constraint is that the edentulous option can be offered only from the SomnoDent^{®} Flex or SomnoDent^{®} Classic or SomnoDent^{®} MAS for users with no upper teeth (in the maxilla), which implies that existence of lower teeth (in the mandible) is necessary. In general, it is recommended that as a prerequisite, a user should have six lower teeth (3-3) in the mandible (as per product brochure SomnoDent-Edentuluos-MAS-Brochure.pdf).

Therefore, there is a need for a MAD that can be used by individuals who are without full natural teeth or who have only partial natural teeth and who suffer from snoring and obstructive sleep apnea.

This background information is provided to reveal information believed by the applicant to be of possible relevance to the present invention. No admission is necessarily intended, nor should be construed, that any of the preceding information constitutes prior art against the present invention.

### SUMMARY OF THE INVENTION

An object of the present invention is to provide a snoring and obstructive sleep apnea prevention device. In accordance with an aspect of the present invention, there is provided a system for preventing or reducing snoring or obstructive sleep apnea in a patient by controlling an amount of mandibular advancement of a mandible of the patient relative to a maxilla of the patient, the system comprising: a mandibular part adapted for fitting engagement with one or more natural teeth, implants and/or gums on the mandible of the patient; a maxillary part adapted for fitting engagement with one or more natural teeth, implants and/or gums on the maxilla of the patient; and an adjustable strap assembly configured to link the mandibular part and the maxillary part to control amount of mandibular advancement of the mandible relative to the maxilla, the adjustable strap assembly comprising: a strap having a first end and a second end; and a pair of casings, each said casing having an axial opening configured to receive a respective one of said first and second ends of the strap, and a lateral groove configured to receive a means for securing the strap in the axial opening of the casing at a desired position; wherein the casings of the adjustable strap assembly are configured to connect to the mandibular part and the strap is configured to connect to the maxillary part, and wherein the length of the strap assembly is adjusted by adjusting the position of the strap within the casing.

In accordance with another aspect of the present invention, there is provided a modular breathing assist device for preventing or reducing snoring or obstructive sleep apnea in a user, the device comprising: a valve body comprising a plurality of openings; a housing configured to receive the valve body in a first position and a second position, said housing having openings that partially or fully align with the plurality of openings in the valve body when the valve body is rotated between the first position and the second position; a mask configured to receive the housing; wherein when said valve body, mask, and housing assembled are assembled, the device is configured for placement in the front portion of an oral cavity of the user to facilitate control of quantity of air flow from mouth during exhaling from the airway through the oral cavity of the user.

In accordance with another aspect of the present invention, there is provided a method of using a system of the present invention to prevent or reduce snoring or obstructive sleep apnea in a patient, the method comprising the steps of: engaging the mandibular part with one or more natural teeth, implants and/or gums on the mandible of the patient; engaging the maxillary part with one or more natural teeth, implants and/or gums on the maxilla of the patient; connecting the mandibular part and the maxillary part using the adjustable strap assembly by attaching each said casing to a rear portion of the mandibular part and snapping the strap into a notch located at a front portion of the maxillary part; adjusting the effective strap length by moving the strap within the casing to achieve adequate mandibular advancement to prevent or reduce snoring or obstructive sleep apnea while considering tolerance and comfort of the patient; and when desired strap length is achieved, tightening the screw to lock the strap within the casing.

### BRIEF DESCRIPTION OF THE DRAWINGS

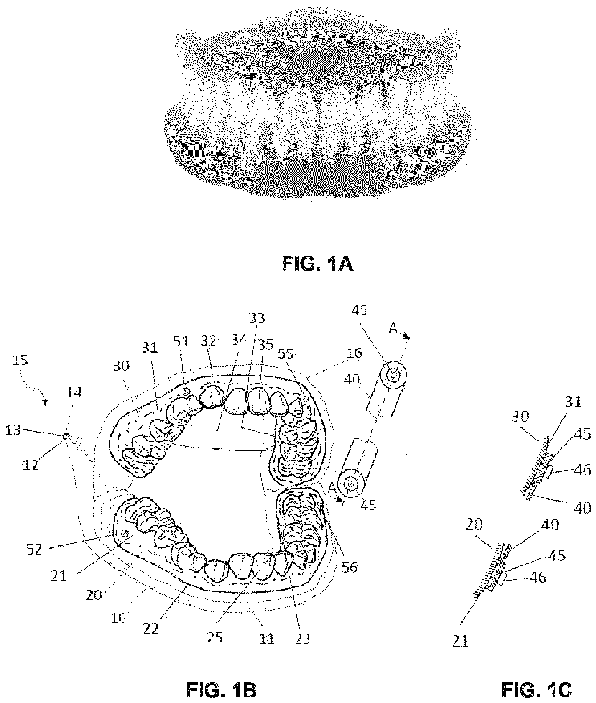

FIG. 1A presents a photographic view of a typical conventional full denture set.
FIG. 1 shows an overall assembled view of an embodiment of the invention having a maxillary full denture, a mandibular full denture, the two connected by a pair of straps.
FIG. 1B presents a view of a typical strap connecting a maxillary denture, and a mandibular denture.
FIG. 1C shows a sectional view of the FIG. 1B.
FIG. 2A shows a representative set of straps of different lengths to enable different distances of mandibular advancement.
FIG. 2B, 2C, 2D show alternative embodiments of configurations of the straps and shapes of pin heads that enable advantages in ensuring securing and ease of change of straps.
FIG. 2E shows a sectional view of a typical multi-layer strap configuration with a stronger cord material reinforcing as core to enable improved durability with flexibility.
FIG. 3 shows a punch and die set for blanking process as an optional manufacturing process for straps to enable economy of scale.
FIG. 4 shows an overall assembled view of an embodiment of the invention having a typical ISD set comprising of a maxillary part, a mandibular part, and a pair of straps connecting the maxillary and mandibular parts to enable desired mandibular advancement.
FIG. 5 shows the schematic diagram for an optional additional production and business process offering a secondary discounted priced unit to a patient ordering a primary denture generating add-on revenue.
FIG. 6 shows an overall assembled view of an embodiment of the invention having an adjustable strap sub-assembly for connecting the maxillary and mandibular parts to enable desired mandibular advancement. Additionally, the figure shows a modular breathing assist device attached to the frontal part of the strap.
FIG. 7 illustrates an exploded view of the components comprising the adjustable strap sub-assembly.
FIG.8A illustrates an enlarged view of a part of the adjustable strap assembly called casing. FIG. 8B illustrates a view from the rear of the view shown in FIG. 8A.
FIG.9 shows the detailed configuration of the strap.
FIGs. 10A-C show a lateral view of the adjustable strap assembly (FIG. 10A), a sectional view A-A (FIG. 10B), and an enlarged cross sectional view of area C (FIG. 10C) consisting of a screw, nut and snap ring.
FIG. 11 illustrates an example of deployment of the adjustable strap on a set of mandibular denture placed on a model of a patient's mandibular jaw and a maxillary part of anti-snoring device worn over a model of natural maxillary teeth resulting in mandibular advancement.
FIGs. 12A-C illustrate an exploded view of the modular breathing assist device components comprising of a valve (FIG. 12A), housing FIG. 12B), and a mask (FIG. 12C).
FIGs. 13A-C illustrate the front view of modular breathing assist attachment (FIG. 13B), a Sectional side view A-A (FIG. 13A) and a sectional top view B-B (FIG. 13C).
FIG. 14 illustrates an enlarged view of a sample embodiment of valve component.
FIG. 15 illustrates a perspective view of housing component of breathing assist device
FIGS. 16A and 16B illustrate two alternate orientations of the valve enabling different quantities of air flow.
FIG. 17A illustrates a clasp option to clamp and hold the breathing assist device on the main body of an anti-snoring device.
FIG. 17B illustrates a free-standing breathing assist device that may be worn by a patient to control breathing exhaust ait flow quantity in conjunction with or without the main body of an anti-snoring device.
FIG. 17C illustrates another additional version embodiment of a breathing assist device housing wherein the inner side of the housing has two receptor brackets with slots for connecting with the front part of strap for fixation on to an anti-snoring device.
FIG. 18 depicts assembly of adjustable strap with breathing assist device housing using the receptor brackets as stated in FIG. 17C.
FIG. 19 illustrates a mask that is attached to the housing and is placed between the frontal dentition and inner surfaces in proximity of a patient's lips.

### DETAILED DESCRIPTION OF THE INVENTION

The present invention provides snoring and sleep apnea prevention devices that are suitable for use by individuals who need full or partial dentures. By providing features in these denture devices that either embedded integrally or as add-on optional accessories augment dentures into preventive snoring avoidance and thus a first line of means to avoid early OSA onset or suffering, the present invention provides a pioneering solution not currently available in the market.

The present invention addresses the needs of this market segment that addresses the annoyance of snoring noise creation, but also safeguards one from OSA, whether or not diagnosed. It may be noted that all currently available MADs in market described in the earlier section of this disclosure are worn over the natural teeth, and thus the field is wide open without any competitive product that can be worn by this niche market segment.

Therefore, an object of the invention is to fill a gap in the field by providing a solution to a substantial number of sufferers from snoring and obstructive sleep apnea without full natural teeth or who have partial natural teeth.

The preferred embodiment of disclosed invention provides an add-on optional feature in a denture of a user to facilitate adjustable or titratable mandibular advancement assisting in partially or fully reducing snoring and thus ameliorating obstructive sleep apnea. The add-on optional feature in the denture set maybe provided in either a regular denture without implants, such as Removable Partial Dentures (RPDs), Removable Full Dentures (RFDs); or in any of the Implant Supported Dentures (ISDs), viz. single tooth dental implant, multiple-tooth on implant-supported bridge, and multiple-tooth on implant-retained denture. Dentures are also called denture prosthesis.

According to one aspect of the invention, the denture set may be provided with an optional set of pins or other means to hold a mandibular part of a denture in an advanced position relative to a maxillary part of the denture using a set of straps of different lengths. Additionally, and optionally, the straps may have a slit or recess to allow relative movement between the mandibular and maxillary parts of the denture.

Courting to another aspect of the invention, optionally, but in addition, the straps may be made of light weight flexible bio-compatible material such as Nylon-12 or a stretchable bio-compatible elastomer allowing relative movement between the mandibular and maxillary parts of the denture in multiple directions of 3D space, viz. forward-backward, upward-downward, leftward-rightward. The straps may be manufactured by several alternative means such as molding, 3D printing, blanking or stamping or other methods that may be available in the state-of-the-art.

Courting to yet another aspect of the invention, optionally, but in addition, the straps may be made of multi-layer hybrid construction wherein a stronger material such as Kevlar^{®} (DuPont^{®}, Wilmington, Delaware, USA) cord is the core to provide strength over which bio-compatible material such as Nylon-12 is coated allowing a combination of both strength as well as flexibility in the relative movement between the mandibular and maxillary parts of the denture in multiple directions of 3D space, viz. forward-backward, upward-downward, leftward-rightward. The multi-layer construction may be achieved by several alternative manufacturing means such as molding, 3D printing, or other methods that may be available in the state-of-the-art.

According to an additional but optional aspect of the invention, the preliminary impression of a patient's mouth obtained by a dentist or denturist by applying pressure to the soft tissues to simulate biting force and extending the borders of the mold to adjacent toothless areas to allow the dentures to better adapt to the gums, usually done in wax to prepare a diagnostic cast or a final cast made of gypsum or other suitable material may be gainfully utilized for the dual purpose of making a denture set as well as a splint set for mandibular advancement.

According to another additional but optional method of use of the invention, the configuration of the patient's denture available with a dentist or denturist may be gainfully utilized for the add-on purpose of making an optional light-weight splint set for mandibular advancement providing a preventive measure for avoiding snoring and thus ameliorating obstructive sleep apnea for those willing to wear denture during sleep.

According to yet another additional but optional embodiment of the invention, an approximate configuration of a patient's natural teeth or denture partially may be adequate to be utilized for the purpose of making a splint set for mandibular advancement deploying a clamp-on approach instead of a complete-fit approach providing a preventive measure for avoiding snoring and thus ameliorating obstructive sleep apnea.

According to yet another additional but optional embodiment of the invention, an adjustable strap configuration is disclosed that can mimic telescopic contraction and expansion, avoiding the need for providing a set of straps.

Courting to yet another aspect of the invention, optionally, but in addition, a modular breathing assist device is disclosed that may not only be attached to the mandibular advancement device embodiments disclosed hereby but also to any other oral appliances. The device has a feature wherein valves to control the quantity of exhaled air do not need to be changed, but a single valve is adjustable to facilitate variation in air flow facilitating additional EPAP therapy complementary to airway opening by mandibular advancement.

In summary, the disclosed invention of snoring and obstructive sleep apnea prevention device offers considerable improvement and advantages over those described in prior art in general, as an optional accessory for dentures in particular.

The invention will now be described with reference to specific examples. It will be understood that the following examples are intended to describe embodiments of the invention and are not intended to limit the invention in any way.

An overall illustration of a conventional full denture set is presented in a photographic view of a typical set in Fig. 1A. The set is well known in the art as upper or maxillary denture and lower or mandibular denture. Each denture has a denture base, a denture flange, denture borders and denture teeth.

Fig. 1 illustrates a configuration of mandibular and maxillary dentures in relation with the lower and upper jaws of a user respectively, pointing out the locations of different anatomical parts of interest in view of the disclosed inventive aspects for fixation of straps to realize mandibular advancement to mitigate snoring or OSA. The jaws of user are shown in an open state to be able to view the internal parts of oral cavity of the user. The lower jaw has lower gum (also called gingiva) part **10** placed over a bone structure **11** in the mandible. The bone structure extends to the back as a Temporomandibular Joint (TMJ) **15** comprising of parts known in the art as condyle **12,** fossa **13,** and disc **14.** The mandibular denture **20** is placed over the lower gum **10** in a reasonably tight but comfortable fit and remains retained during day-to-day activities of the user such as speaking, chewing, etc. In a standard denture that does not use any implants to secure the denture to the mandible, the shape and fit between the mandible and the mandibular denture serve the purpose of retention.

The user is generally provided with options for increased levels of retention, for example, by inserting or applying between denture **20** and gum **10** bio-compatible human use safe glue materials claiming several characteristics such as being Zinc free, impervious to water, providing soft cushion, quality seal from food particles, ooze-control, use of natural ingredients like gum wax, beneficial herbs, and vitamins. Several glue products are available, for example, Effergrip^{®} denture adhesive cream (manufacturer: Prestige Consumer Healthcare, Irvington, New York; Source: Amazon^{®} USA), Fixodent^{®} Ultra Max Hold Dental Adhesive (Procter & Gamble^{®}, Cincinnati, OH, USA), SECURE^{®} Sensitive Adhesive (Cutting Edge International, LLC, Los Angeles, CA, USA), Instant Smile^{™} Secure Fit Adhesive (Billy Bob Products Inc., Hardin, IL, USA), Super Poligrip^{®} Denture and Partials Adhesive Cream (Glaxo Smith Kline, Philadelphia, PA, USA), Corega^{®} Denture Adhesive Cream (Glaxo Smith Kline, Bulgaria, Source: Amazon^{®}, USA), Y-Kelin Denture Adhesive Cream (Anhui Greenland Biotech Co. Ltd., Bengbu, Anhui, China), DenSureFit^{®} Lower Denture Reline Kit (OTC Dental Inc., Vancouver, WA, USA), and from many other manufacturers or sources.

The use of glue between denture and gum is found to achieve good average retention force in compliance with reported research, for example, in the range of 16.66 ± 7.32 lbs for milled denture bases and in the range of 12.19 ± 6.15 lbs for the conventional heat polymerized denture bases (AlHelal, Abdulaziz Abdullah, "Comparison of Retention between Milled and Conventional Denture Bases: A Clinical Study" (2016), Loma Linda University Electronic Theses, Dissertations & Projects, 323). Our experimental tests have shown that 3D printed dentures provide better or at least comparable retention force relative to milled denture bases. It has been found that this retention force is adequate to withstand the mandibular advancement force reported in the art as approximately 1 to 1.2 Newtons per millimeter of mandibular advancement (for example, 1.18 Newtons per millimeters as per J Cohen-Levy, B Pételle, J Pinguet, E Limerat, B Fleury, Sleep Breath, 2013 May;17(2):781-9. doi: 10.1007/s11325-012-0765-4. Epub 2012 Sep 11).

The mandibular denture **20** comprises of an exterior wall or flange **21,** an exterior ridge or border **22,** and an interior ridge or border **23.** A cavity known in the art as trough is formed between the exterior and interior walls that fits over the lower gum **10.** A set of teeth **25** that substitute natural teeth known in the art with nomenclature (starting from back or TMJ side, a set of teeth- one on left and one on right sides) Second Molar, First Molar, Second Premolar, First Premolar, Canine or Cuspid, Lateral Incisor, and Central Incisor (front most teeth set) are embedded symmetrically in the mandibular denture in a U-shaped configuration. This is considering the prevailing trend of not having Third Molar or Wisdom teeth at the back most location, like the natural teeth set where their extraction is recommended by dentist profession, resulting in a total fourteen teeth in the mandibular denture.

Analogous to the mandibular denture **20,** the maxillary denture **30** comprises of an exterior wall or flange **31,** an exterior ridge or border **32,** and an interior ridge or border **33.** A cavity known in the art as trough is formed between the exterior and interior walls that fits over the upper gum **16.** Generally, an impression of upper palate of user's oral cavity is obtained to provide a bridge between the left and right sides of interior walls in the maxillary denture. This bridge part known as Palate **34** generally is in close contact with the user's upper palate helping in improved retention of the maxillary denture. The maxillary denture **30** is placed over the upper gum **16** in a reasonably tight but comfortable fit and remains retained during day-to-day activities of the user such as speaking, chewing, etc. In a standard denture that does not use any implants to secure the denture to the maxilla, the shape and fit between the maxilla and the maxillary denture along with palatal contact serve the purpose of retention. The user is generally provided with options for increased levels of retention, for example, by inserting or applying between denture **30** and gum **16** bio-compatible human use safe glue materials claiming several characteristics as mentioned earlier in the context of mandibular denture. A set of teeth **35** that substitute natural teeth known in the art with nomenclature (starting from back or TMJ side, a set of teeth- one on left and one on right sides) Second Molar, First Molar, Second Premolar, First Premolar, Canine or Cuspid, Lateral Incisor, and Central Incisor (front most teeth set) are embedded symmetrically in the maxillary denture in a U-shaped configuration. This is considering the prevailing trend of not having Third Molar or Wisdom teeth at the back most location, like the natural teeth set where their extraction is recommended by dentist profession, resulting in a total fourteen teeth in the maxillary denture.

A location **51** on the user's right-side wall **31** of the maxillary denture **30,** and location **52** on the user's right-side wall **21** of the mandibular denture **20** exemplify the locations of connection of pins **45** illustrated in Fig. 1B and Fig. 1C. Similarly, a location **55** on the user's left-side wall of the maxillary denture **30,** and location **56** on the user's left-side wall of the mandibular denture **20** exemplify the locations of connection of pins **45** illustrated in Fig. 1B and Fig. 1C. The pins may be integrated structurally with respective denture body while manufacturing the same by molding, milling, 3D printing or additive manufacturing or other fabrication methods. Alternatively, other approaches also may be deployed such as affixing the pins into the denture bodies by adhesion, fitting into receptor holes, or by screwing into receptor threaded holes by having matching threads in the pins, or a combination of such approaches. Fig. 1B illustrates a view of a strap **40** assembled with the dentures using the pins **45** as viewed from the front of the walls of the dentures.

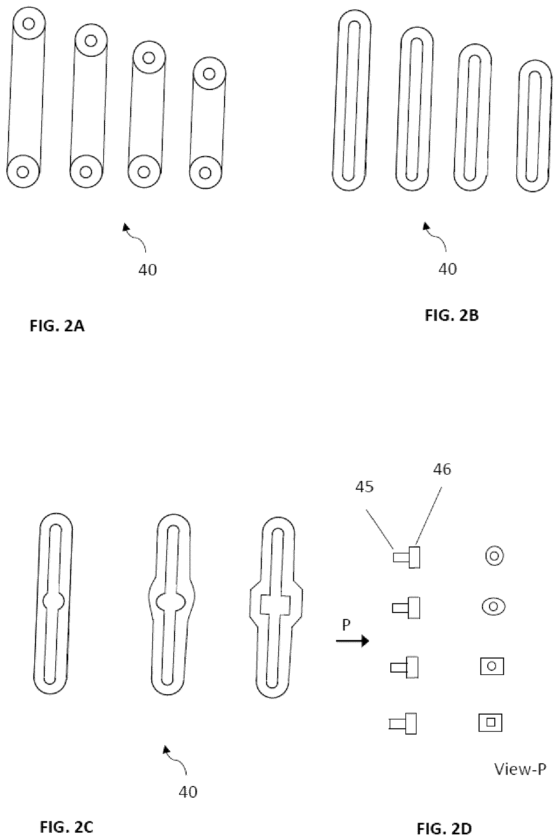

Fig. 1C illustrates a sectional view of the assembly shown in Fig. 1B wherein the strap **40** is retained or fixed on the maxillary denture **30** and mandibular denture **20** using a pair of pins **45** that have pin heads **46.** The pins and pin heads are not sectioned in the section A-A view shown in Fig. 1C. The pin heads **46** may be an integral part of the pins **45** in an embodiment of the invention. The pin heads **46** may be attached to the pins **45** in another optional embodiment of the invention by means such as threads, snap fit, and other methods. The shapes of the pins and the pin heads are shown as circular cylindrical in the embodiment illustrated in the Figs. 1B and 1C, however, those skilled in the art may appreciate that there are several other conceivable shapes possible, and all such shapes are included in the spirit of this invention. The gainful deployment of a few of those shapes are illustrated in Fig. 2D and described in the following paragraphs.

Fig. 2A illustrates a set of 4 different lengths of strap **40.** The set of different lengths enables customization of the amount of mandibular advancement that may work most effectively for a particular user. Typically, the relative positions of pins **45** fixed on the mandibular denture **20** and maxillary denture **30** is such that use of shortest strap results in maximum mandibular advancement, whereas the use of longest strap results in minimum mandibular advancement. Different strap lengths may be tried between these to optimise the amount of mandibular advancement best suited for a particular user considering factors such as clearance of airway passage, and comfort of use including reduction or management of side effects such as temporomandibular joint (TMJ) pain. Optionally and preferably, the configuration of a strap 40 illustrated in the Figures has a relatively thicker boss area surrounding the pin hole to provide better strength and thus resistance to tear due to the mandibular advancement force reported in the art as approximately 1 to 1.2 Newtons per millimeter of mandibular advancement (for example, 1.18 Newtons per millimeters as per J Cohen-Levy, B Pételle, J Pinguet, E Limerat, B Fleury, Sleep Breath, 2013 May;17(2):781-9. doi: 10.1007/s11325-012-0765-4. Epub 2012 Sep 11, as quoted earlier). Those skilled in the art may appreciate that there may be other number of straps in a set ranging from 2 to 10 or more or even a single strap optimised a priori before handing over to a user, and all such possibilities are within the scope and spirit of this invention disclosure.

Fig. 2B illustrates a set of 4 different lengths of another optional configuration of strap **40** wherein there is a slot. The set of different lengths enables customization of the amount of mandibular advancement that may work most effectively for a particular user. Typically, the relative positions of pins **45** fixed on the mandibular denture **20** and maxillary denture **30** is such that use of shortest strap results in maximum mandibular advancement, whereas the use of longest strap results in minimum mandibular advancement. Different strap lengths may be tried between these to optimise the amount of mandibular advancement best suited for a particular user considering factors such as clearance of airway passage, and comfort of use including reduction or management of side effects such as temporomandibular joint (TMJ) pain. Optionally and preferably, the configuration of a strap 40 illustrated in this Figure has a slot recess connecting the pin holes to provide sliding between the pin and the slot thus better flexibility of movement between maxillary and mandibular jaws in forward-backward, upward-downward, and lateral (leftward-rightward) directions. Those skilled in the art may appreciate that there may be other number of straps in a set ranging from 2 to 10 or more or even a single strap optimised a priori before handing over to a user, and all such possibilities are within the scope and spirit of this invention disclosure.

Optionally, and in addition to the configurations described so far, Fig. 2C illustrates a circular, elliptical, and a rectangular portion of increased sized recess or slot portion at the middle portion of the strap. This larger recess in the slot of strap is intended to allow a circular, elliptical or rectangular head **46** of pin **45** to come out of the strap facilitating ease of change of strap. This configuration of the invention is incorporated to ameliorate the problem of unwanted disengagement between pin and strap when the pin hole or slot connecting the pin holes is kept too large to facilitate the ease of change of strap. By providing a larger recess only at the middle part, the disengagement during use inside mouth of the user is avoided. As the strap is required to be changed only outside the mouth of user, the maxillary and mandibular dentures can be freely moved to a relative position such that a pin can access the wider recess at the middle of a strap to facilitate removal of strap from pin. The figure shows increase in outer width of the strap to retain strength of the strap at the middle portion.

Fig. 2D illustrates the different shapes of pin heads **46,** viz. circular, elliptical and rectangular cross-sections, as can be seen in end view-P, whereas, the pin is generally circular cross section in most cases.

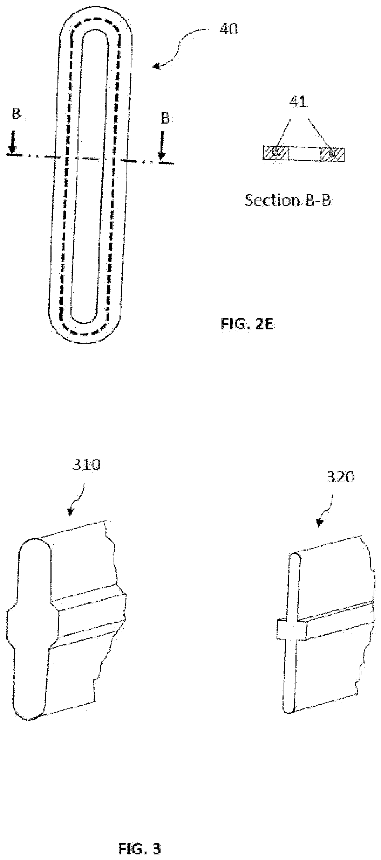

An optional but additional configuration of the strap has a stronger reinforcement cord embedded inside a relatively weaker strap material to solve a problem that was encountered during trials, namely tearing off or severance of the strap. This is of particular relevance and advantage of this disclosure where the strap is made of flexible material, for example, made of one of the acceptable bio-compatible elastomers (Saliterman, Steven S., "Introductory Medical Device Prototyping: Medical Device Polymers", Presentation, Dept. of Biomedical Engineering, University of Minnesota, USA); whereas the reinforcement cord may be made of a stronger material such as Kevlar^{®} (DuPont^{®}, headquartered at Wilmington, Delaware, USA, manufactured by Advanced Fibre Systems, VA, USA). Fig. 2E (not to scale, only indicative representation) shows a sectional view of a typical multi-layer strap configuration with a stronger cord material reinforcing as core to enable improved durability with flexibility. The circular cross section of the reinforcing cord **41** can be seen embedded inside the strap cross-section. The combination of both strength as well as flexibility allows the relative movement between the mandibular and maxillary parts of the denture in multiple directions of 3D space, viz. forward-backward, upward-downward, leftward-rightward. The multi-layer construction may be achieved by several alternative manufacturing means such as molding, 3D printing, or other methods that may be available in the state-of-the-art, gainfully deployed for this application.

Mass production of straps deploying economy of manufacture scale may be an optional yet another additional method deployed as an alternative to a stronger reinforced strap configuration. Fig. 3 illustrates a perspective view of configuration of a typical punch set showing the contours of the end faces of the punches **310** and **320** that may be deployed for progressive 2-step blanking process, each using a different punch and die set (dies are not shown in the figure) as an optional manufacturing process for straps (for example, the third shape out of the shape options illustrated in Fig. 2C) to enable economy of scale, wherein a large number of inexpensive spare straps may be provided instead of stronger and costlier straps. It can be appreciated by those skilled in the art that a punch-die set comprises of closely slidably fitting cavity in a die that receives a correspondingly shaped protrusion known as punch, the relative impact between die and punch piercing the part of a sheet material of interest placed between a punch and a die. In the illustrated case, the pierced part produced by the first set of punch-die interaction will produce an interim part as blank, while the second set of punch and die interaction will produce a useful intended strap part and a scrap or waste part corresponding to the shape of slot or recess in the strap. Bio-compatible versions of materials such as Kapton^{®} (DuPont^{®}, headquartered at Wilmington, Delaware, USA) available readily in sheet form are suitable raw material for such blanking process.

While the figures and description in this disclosure has presented in detail full maxillary and mandibular dentures, several additional and optional embodiments have been developed that are covered by the scope and spirit of the overall invention. For example, an embodiment of the invention has a typical RPD set comprising of a maxillary part, a mandibular part, an embrasure clasp, a pair of multiple circlet clasps, and a pair of straps connecting the maxillary and mandibular parts to enable desired mandibular advancement. Clasps are means to secure partial dentures with the help of surviving natural teeth in a person's jaws. The terminology of different types of clasps are well known in the denture related art, but to our knowledge not gainfully deployed or used in oral appliances for obstructive sleep apnea or anti-snoring devices. It has been found that the forces that can be withstood by unique designs and arrangements of different types of retainers/ clasp assemblies, without glue, to have enough retention force to withstand mandibular advancement force that is in fact quite small. This is found to be valid for several clasp designs, for example, Circumferential (Circle or Akers) clasp, Ring clasp, Embrasure (Double Akers) Clasp, "C" clasp (Hair-pin or Reverse action), etc.

Another optional variant of the embodiment described above is the one wherein an improvised embrasure clasp is deployed to enable facilitation of resting the tongue of wearer constraining it so that it does not slide backwards contributing to airway blockage.

In yet another optional and additional variant of the embodiment discussed so far, a combination of multiple improvised clasps is deployed to enable facilitation of adjustable mandibular advancement with or without the need for straps.

Furthermore, in another optional variant of the embodiment presented above, a combination of multiple improvised clasps is deployed to enable facilitation of adjustable mandibular advancement without the need for straps, additionally providing for resting the tongue of wearer constraining it so that it does not slide backwards contributing to airway blockage.

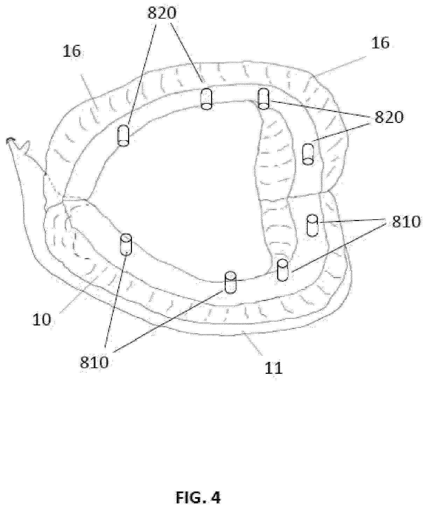

Furthermore, another optional embodiment of the invention has a typical ISD set comprising of a maxillary part, a mandibular part, and a pair of straps connecting the maxillary and mandibular parts to enable desired mandibular advancement. Fig. 4 illustrates typical locations of implant pins **810** on the mandible's lower gum **10** over bone structure **11,** and implant pins **820** on the upper gum **16** of maxilla that are used to retain the denture on the wearer's jaws, essentially serving the purpose of retention of denture without the use of adhesive. The dentures possess mating holes that slidably and snugly receive corresponding implant pins to form good connection and thus retention of dentures over the gums. These dentures are found to have higher retention force capacity compared to adhesive based retention, thus can very easily overcome the mandibular advancement forces. Those skilled in the art can appreciate that the number of implants and their locations depend on several factors such as jawbone density, jawbone loss, bone grafting, etc.; and thus although 2 to 4 implants per jaw are typical, there may be more implants necessary in some cases, for example, 12 to 16 dental implants have been found necessary in some cases. For the purpose of withstanding the mandibular advancement force of approximately 1 to 1.2 Newtons per millimeter of mandibular advancement, considering that the advancement required is generally within 5 millimeters, these numbers of implants are adequate. Generally, the number of implants needed for the dual purpose of general denture retention for chewing and for mandibular advancement is found to be same or close to the number of implants needed for single conventional purpose of denture retention only for chewing.

Furthermore, in another optional but additional variant of the embodiment described above, an improvised embrasure clasp is deployed to enable facilitation of resting the tongue of wearer constraining it so that it does not slide backwards contributing to airway blockage.

Furthermore, in yet another optional but additional variant of the embodiment described above, a combination of multiple improvised clasps is deployed to enable facilitation of adjustable mandibular advancement with or without the need for straps.

Furthermore, in yet another optional but additional variant of the embodiment described above, a combination of multiple improvised clasps is deployed to enable facilitation of adjustable mandibular advancement without the need for straps, additionally providing for resting the tongue of wearer constraining it so that it does not slide backwards contributing to airway blockage.

Additionally, but optionally, a universal version of the invention utilizing a compliant clasp approach is also invented that may be worn over either denture or natural teeth due to its lightweight and ease of attachment-detachment configuration to facilitate anti-snoring provisions.

Furthermore, in yet another optional but additional variant of the embodiment described above, another universal version of the invention utilizes a compliant clasp approach enabling stepless mandibular adjustment that may be worn over either denture or natural teeth due to its lightweight and ease of attachment-detachment configuration to facilitate anti-snoring provisions.

Furthermore, yet another optional but additional universal version of the invention utilizes a compliant clasp approach that may also be elastic enabling stepless mandibular adjustment with spring characteristic that may be worn over either denture or natural teeth due to its lightweight and ease of attachment-detachment configuration to facilitate anti-snoring provisions with enhanced comfort for the wearer allowing jaw movement.

Furthermore, yet another optional but additional universal version of the invention utilizes a reactive latch mechanism that realizes a mandibular advancement only after the user has gone on sleep causing excessive relaxation of the mandible relative to the maxilla, to facilitate anti-snoring provisions with enhanced comfort for the wearer allowing natural or moderate relaxed jaw positions during onset of sleep.

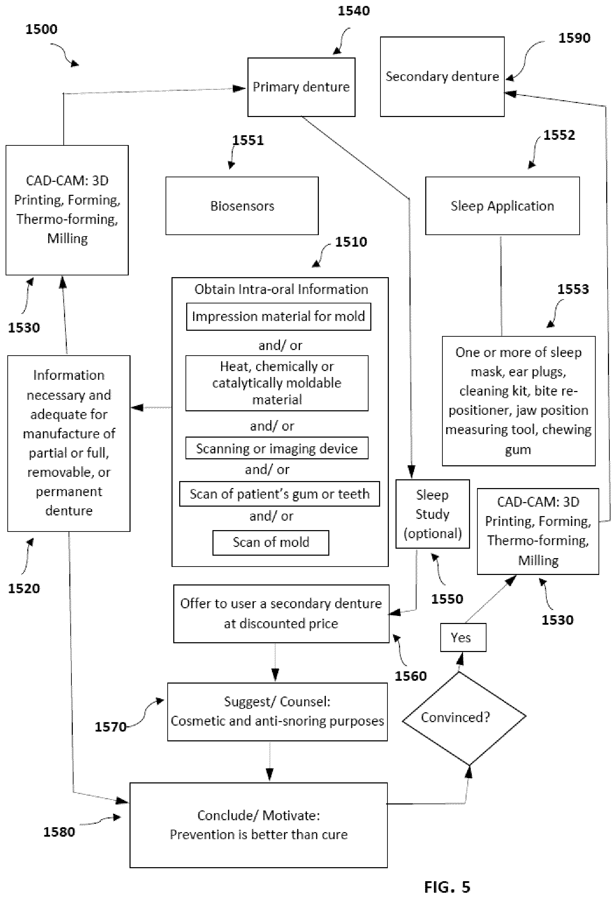

Fig. 5 illustrates the schematic diagram for an optional additional inventive process **1500** comprising of economical production of a secondary unit that may be offered to a patient ordering a primary partial or full, removable or permanent denture; at a discounted price that may serve the purpose of cosmetic function as well as anti-snoring function. This also illustrates gainful deployment of a business process promoting sale of primary denture, and/or add-on revenue generation through sale of secondary denture. This innovative process obtains intra-oral information **1510** that may involve use of either conventional mold material usage such as impression material and allied equipment for capturing the configurations of user's mandibular gum and maxillary gum or adjacent teeth in addition to the gums in the case of removable partial dentures (RPDs). Alternately, heat, chemically, or catalytically moldable materials may be used to obtain the impression. Optionally, other alternatives such as scanning of user's gum or teeth using imaging devices or scan of mold may be resorted to. This information **1520** is necessary and adequate for manufacture of partial or full, removable or permanent denture, and may be gainfully used or deployed for manufacturing either a primary denture **1540** and/or a secondary denture **1590.** The manufacturing process **1530** may include Computer Aided Design (CAD) and Computer Aided Manufacturing (CAM) comprising of different options such as 3D printing, forming, thermo-forming such as injection molding, or milling.

While delivering the primary denture ordered by the user, or before the ordering process, or during the ordering process, or subsequently, an optional sleep study **1550** may be offered to the user. The sleep study may comprise of any of the different levels of sleep studies (Level 1, Level 2, Level 3, Level 4) either at a sleep lab or at home. With or without this optional sleep study, the user of denture may be offered a secondary denture at a discounted price. This offer process step **1560** involves suggestion and counselling **1570** about the advantages of a secondary set of dentures to be worn at night during sleep time that may be either an exact duplicate of the primary denture, or alternatively and preferably; a simpler, lighter, version with provisions such as increased exposure of gums to air as the purpose of this secondary denture is not chewing of food. While serving cosmetic purposes avoiding any embarrassing situations where the user maybe exposed to known or new acquaintances by getting caught off-guard without any denture, the secondary denture also serves the important purpose as an anti-snoring device. Even if the user is not aware or not formally diagnosed for sleep disorders knowingly or un-knowingly, the secondary denture serves an important function of prevention is better than cure motto **1580.** Once the user is convinced of the advantages of acquiring a secondary denture at discounted price, the necessary information **1520** for production that was available earlier for primary denture production may be advantageously re-used for manufacture process **1530** to produce the secondary denture **1590.** While sleep study **1550** is just optional, in case the same is preferred by a user, furthermore optionally, related additional appliances such as a variety of biosensors **1551,** sleep application **1552,** and a kit **1553** comprising of one or more of a sleep mask, ear plugs, cleaning kit, bite re-positioner, jaw position measuring tool, and chewing gum may be offered and deployed for the sleep study. The biosensors **1551** deployed may be one or more biosensors configured to measure one or more sleep parameters, wherein the one or more sleep parameters are selected from heart rate, breathing rate, head movement, body movement, bite force, body temperature, blood glucose levels, actigraphy, oximetry, apnea hypopnea index (AHI), peripheral arterial tone (PAT), cardiac data, chest expansion, respiratory air flow, sleeping position, patient compliance and snoring.

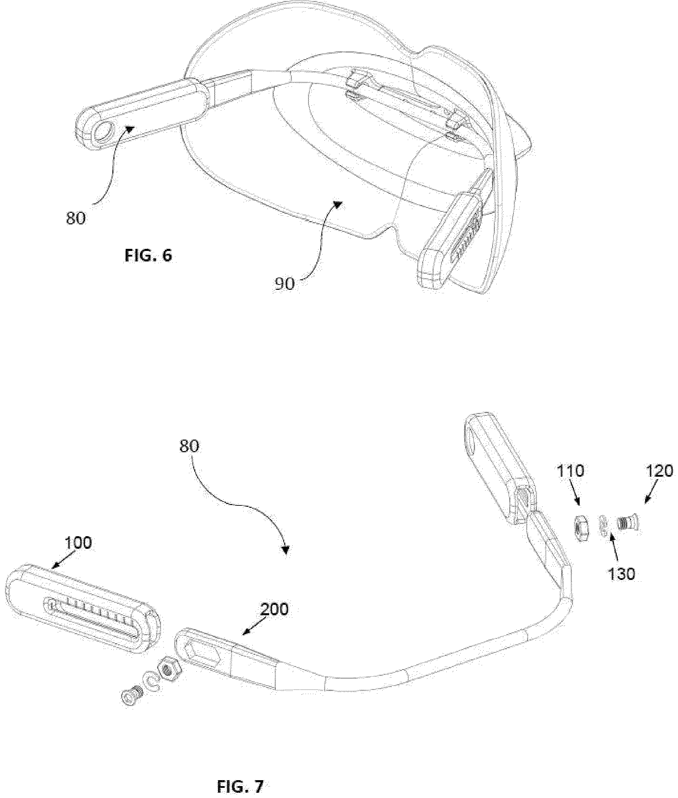

FIG. 6 shows an overall assembled view of an embodiment of the invention having an adjustable strap sub-assembly **80** for connecting the maxillary and mandibular parts of an anti-snoring device to enable desired mandibular advancement. This configuration of strap is an alternative to the straps **40** described earlier, and avoids the need to provide a set comprising of multiple straps. Additionally, the figure shows a modular breathing assist device sub-assembly **90** attached to the frontal part of the strap. The maxillary and mandibular parts of an anti-snoring device to enable desired mandibular advancement may be an upper member for receiving upper teeth of the user (upper splint or tray) structured to engage with one or more natural teeth on user's maxilla and a lower member for receiving lower teeth of the user (lower splint or tray) structured to engage with one or more natural teeth on user's mandible, respectively. The maxillary and mandibular parts of an anti-snoring device to enable desired mandibular advancement may alternatively be an upper full or partial denture structured to engage with gums or engage with one or more surviving natural teeth through clasps or engage with implants on user's maxilla and a lower full or partial denture structured to engage with gums or engage with one or more surviving natural teeth through clasps or engage with implants on user's mandible.

FIG. 7 illustrates an exploded view of the components comprising the adjustable strap sub-assembly. Component **100** is a casing for fixing and adjusting the effective overall length of its assembly with strap **200,** and is also responsible for the connection to the mandibular part of a sleep appliance, by means such as a ball joint. Typically, a pair of casings **100** is deployed, one casing at the right side of the appliance, and one casing at the left side of the appliance. The strap **200** keeps the left and right sides of casings **100** connected.

Nut **110** and screw **120** are two sets of nuts and screws (one set for the left casing and one set for the right casing) for securing/locking **100** and **200.** Component **130** is a C-shaped snap ring whose purpose is to prevent screw **120** from falling off by coming out of the assembly which is a safety concern. The C-shaped snap ring slides into a groove in the screw and acts as a retainer. Those skilled in the art may appreciate that there can be several other means to retain the screw and all such variants are covered under the spirit and scope of this invention.

Furthermore, there can be several means to retain the C-shaped snap ring in place to keep the screw retained avoiding sliding out of the screw from the ring and all such means are covered under the spirit and scope of this invention. As an example, the C-shaped snap ring may be rotated by adequate angle (e.g. 90 degrees) and restricted from easily returning back by its external shape configured as a cam mechanism wherein higher torque is required and that can be exerted only when deliberately needed to disassemble the device. As another example, bio-compatible glue may be applied after sliding the screw into the recess in the C-shaped snap ring, to fill the recess to inhibit the screw from sliding out of the recess.

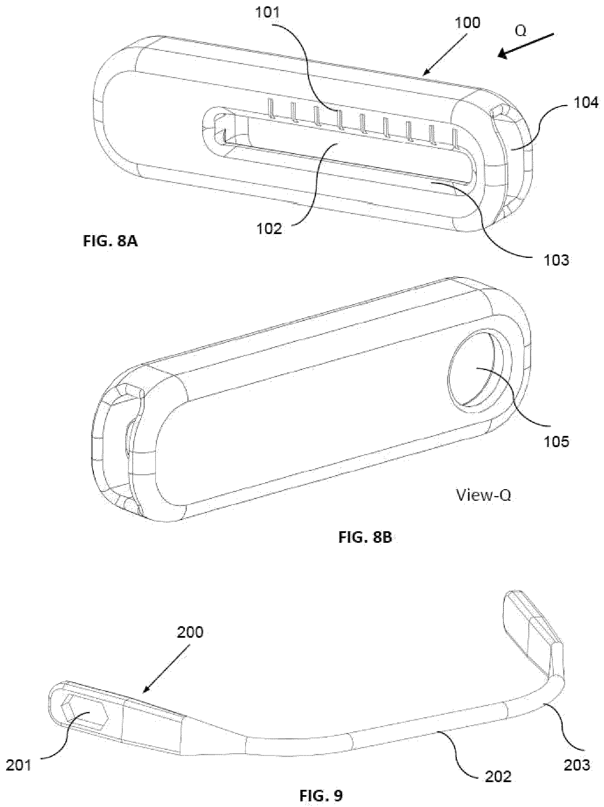

FIG. 8A illustrates an enlarged view of a part of the adjustable strap assembly called casing. FIG. 8B illustrates a view from the rear of the view shown in FIG. 8A. Markings or gradations **101** is a scale for measuring and adjusting the length of the strap. Lateral groove **102** receives the screw **120** and the nut **110,** which will lock onto when the screw is tightened using a tightening tool such as an allen key or screw driver depending on structure of screw head, and as described earlier, the C-shaped snap ring **130** placed behind the wall **103** of the casing blocks the screw **120** from coming off the front of the wall in the casing.

The axial opening **104** in the casing is for the insertion of strap **200.** The spherical shaped cavity **105** is the ball joint receptor part for attachment to the sleep appliances, typically on the rear portion of their mandibular part wherein a button or matching spherical shaped projecting peg is made available for insertion into the cavity.

FIG. 9 shows the strap **200** wherein **201** is a lateral recess for the placement of the nut **110** and the C-shaped snap ring **130.** The recess **201** has additional lateral space to facilitate small amount (typically 2-3 millimeters but larger lengths may be provided in special cases based on user's comfort and preference) of sliding of the strap even when the stopper assembly is locked thus locking the gross length of the strap, as it is found to be more comfortable for the patient while wearing the anti-snoring appliance.

The front part **202** of the strap **200** is typically thinner relative to the lateral parts entering the casings **100** and it connects to the upper or maxillary part of the anti-snoring appliance by snap-in to a tight fitting recess in the maxillary part. As described earlier, there is some room to move the maxillary part forward or backward relative to the mandibular part while wearing the appliance.

The corner part **203** of the strap **200** is typically a rounded corner to avoid discomfort as it is likely to touch the inner surfaces of cheeks near the lips of patient's mouth.

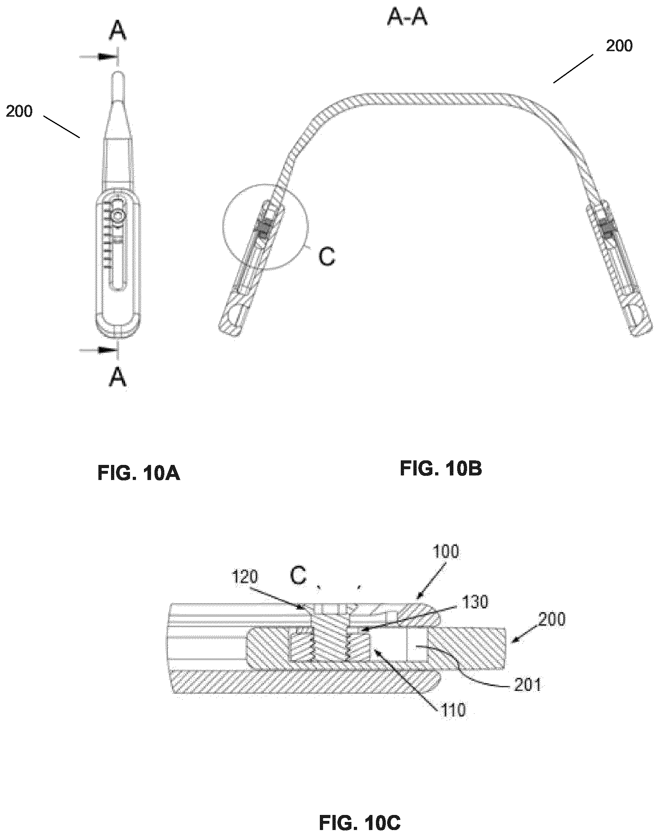

Fig. 10A depicts a lateral view of the strap assembly **200,** and Fig. 10A is a sectional view of the complete assembly **200.** It illustrates how the screws **120** and nuts **110** will lock onto casing **100** when tightening **110** and **120** together with the C-shaped snap ring **130** slided into the groove in the screw **120,** enabling a stopper locked on the casing **100** at a desired position most suitably customized for an individual wearer patient. It can be seen from the enlarged sectional view of area C shown in Fig. 10C that recess **201** in the strap **200** still has some room to enable the strap to move into the casing. This enables the patient wearing the sleep appliance to move the mandibular and maxillary jaws relative to each other a bit even when the stopper assembly is locked.

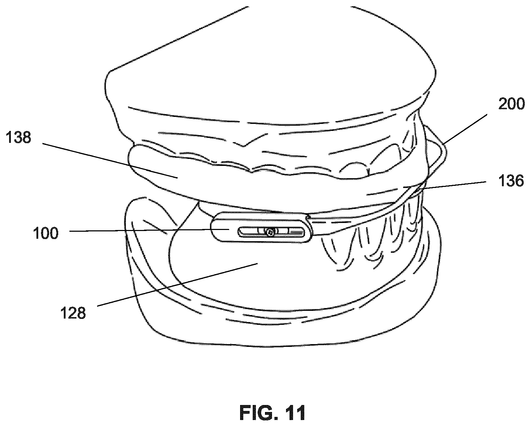

FIG. 11 illustrates an example of deployment of the adjustable strap **200** on a set of mandibular denture **128** placed on a model of a patient's mandibular jaw and a maxillary part **138** of anti-snoring device worn over a model of natural maxillary teeth resulting in mandibular advancement. The maxillary part has a notch **136** wherein front middle part of strap encircling the maxillary part gets retained by means of a snap fitting action. The strap can be snapped out easily for disassembly, when needed. Typically, the adjustable strap could vary the amount of mandibular advancement from 0 to 10 millimeters, but it can be larger if needed in special cases. Effective strap length needed for adequate mandibular advancement estimated for relief from snoring or obstructive sleep apnea while considering user tolerance and comfort is adjusted and subsequently screw is tightened for locking the stopper.

The components of adjustable strap assembly may be made of different bio-compatible materials generally used in the art for oral appliances and dentures including but not limited to Nylon, PEEK (Polyether Ether Ketone), other polymers such as Polyamide, metals used in the art for manufacture of denture frameworks such as Co-Cr, Ti-6Al-4V, and emerging improved materials such as alloys enriched with nano-particles to enhance their properties. The manufacturing processes may also vary depending on scale and quantity of production including additive manufacturing, injection molding, wire drawing, and machining. Furthermore, some parts of the assembly may be manufactured by one method and others may be manufactured by different methods.

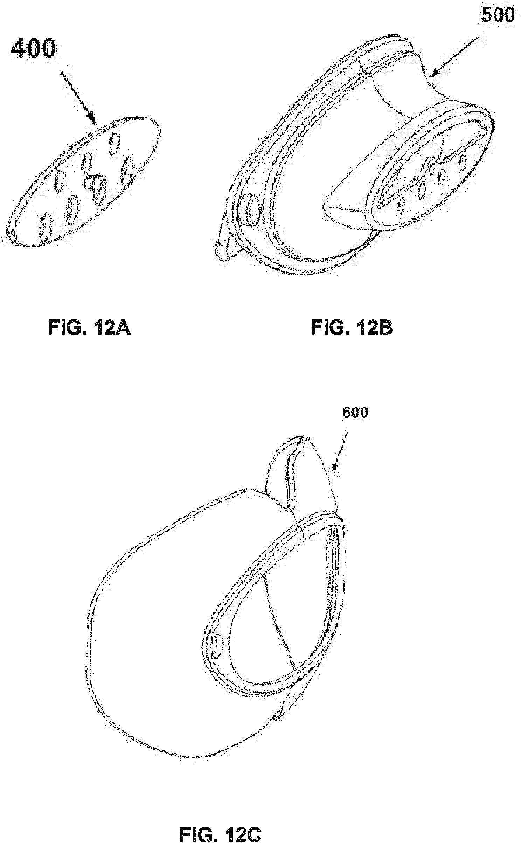

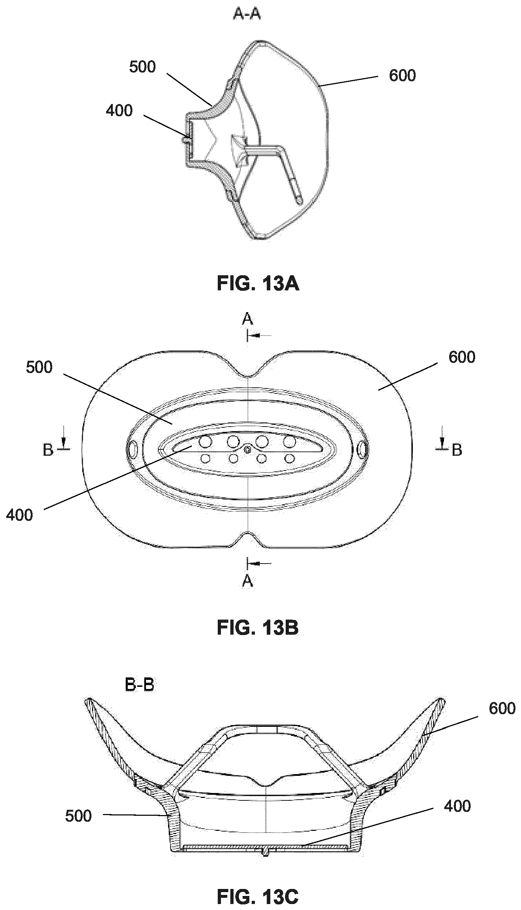

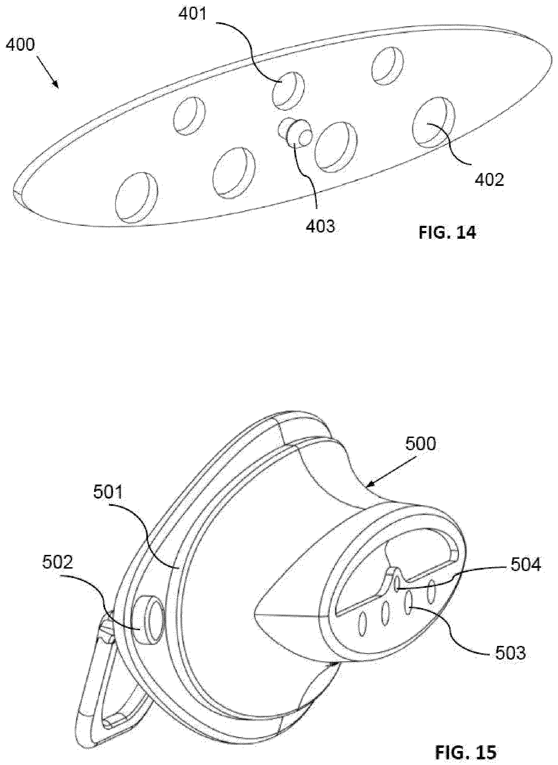

The disclosed modular breathing assist attachment sub-assembly **90** (exploded view shown in FIGs.12A-C, sectional views shown in FIGs. 13A-C) comprises of a valve body **400** (FIG. 14) that is a soft rubber gasket responsible for the control of quantity of air flow from mouth or oral cavity of a user during exhaling from the airway through oral cavity of the user, which is connected to a housing **500** (shown in FIG. 15) by means of a projecting snap button **403** in the center of valve **400** facilitating a snap fit connection. The valve body has holes of different sizes, for example, three relatively small holes **401** in one row and four relatively large holes **402** in another row.

The main function of housing **500** is to connect valve body **400** and mask **600;** and additionally, to play a role in fixing the assembly on a mandibular or maxillary part of an anti-snoring device. Groove **501** on the outer periphery of **500** is designed to connect with close fit with inner mating cavity **601** of mask **600.** Button **502** is a projected peg which serves as a receptor to fix mask **600** on the housing and prevent mask **600** from slipping off or rotating. Housing is made of bio-compatible Nylon, PEEK, or rubber and made by using 3D printing or additive manufacturing, or alternatively by injection molding.

The receptor hole **504** in the housing **500** is connected to the snap button **403** of the valve body **400** enabling holding of the same in place and because of its cylindrical configuration, allows the valve body **400** to be rotated between a first position and a second position for adjustment of the quantity of air flow as described earlier. The holes **503** fully or partially align with some of the holes **401** or **402** of the valve body controlling the quantity of air flow by varying the angle of rotation of the valve body **400** between a first position and a second position in relation with the housing **500.**

Due to the asymmetrical design of the top and bottom parts of **400,** the user can rotate the valve **400** (e.g. by 180 degrees) thereby adjusting the flow rate of the air for breathing out, and the difference in the size of the openings **401** and **402** can vary the flow rate of air for breathing out or exhaling from the airway through oral cavity of user. Several locking means are deployed after rotation of valve. Varying the rate of flow of exhaling air creates difference in resistance to outflow of air from a wearer's oral cavity, which in turn assists in opening of the airways.

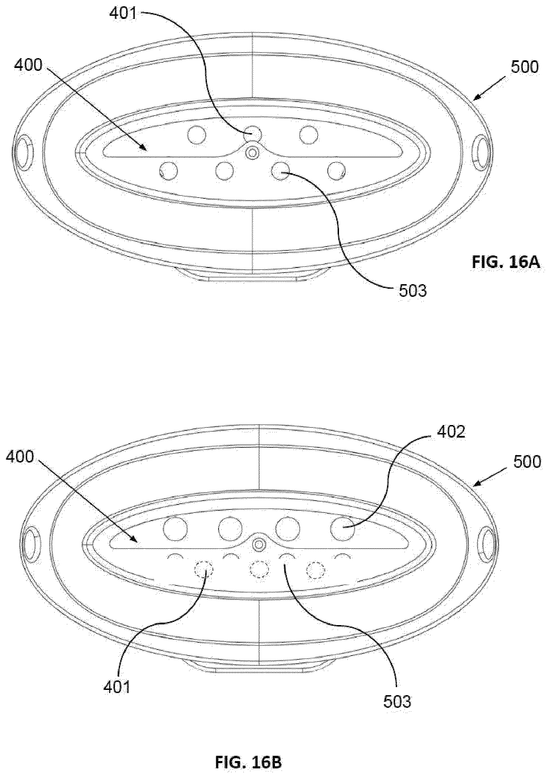

As an example, in FIG. 16A, the openings **503** in housing **500** are designed to adjust the flow of air for breathing out; openings **503** working with openings in valve body **400.** When valve **400** is used by rotating it in a direction and to an extent such that openings **401** are facing up, all of its 7 holes (four holes **402** aligned with **503,** and three holes **401**) can circulate the air.

However, as another example, in FIG. 16B, when valve **400** is used by rotating it in a direction and to an extent such that openings **402** are facing up, because of the effect of its mismatch, valve **400** will block the four holes **503** in the housing **500,** simultaneously the valve **400**'s three holes are blocked by the housing **500,** and thus the air will only flow out through four holes **402** in the valve **400.** Those skilled in the art may appreciate that there can be several variants of this design, for example by changing the number of openings exposed, partial or full exposure of certain openings, different sizes of openings, locking means, etc.; and all those variants are covered by the spirit and scope of this disclosure.

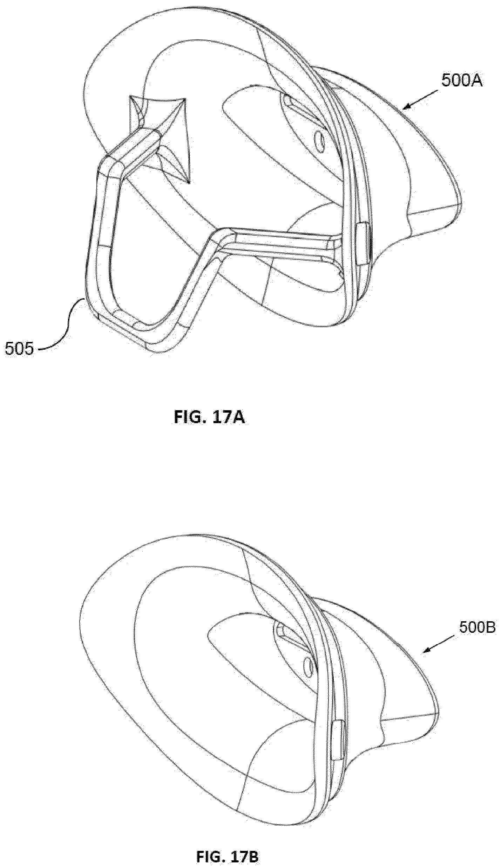

In one version of embodiment of the housing (FIG. 17A), namely, **500A,** the rear side of housing component **500A** has a C-shaped clasp configuration comprising of connecting bars **505** made of a bio-compatible material such as Nylon 12 or PEEK with a certain degree of flexibility, necessitated by the preferable optional feature that the disclosed modular breathing assist can be used in combination with most sleep appliances available in the market in addition to the anti-snoring device disclosed.

Another embodiment variant **500B** shown in FIG.17B relies on the shape and fitting characteristics of housing **500** and mask **600** to maintain its position in the wearer's mouth or oral cavity between lips and dentition and can be used as stand-alone device even when not wearing other oral appliances.

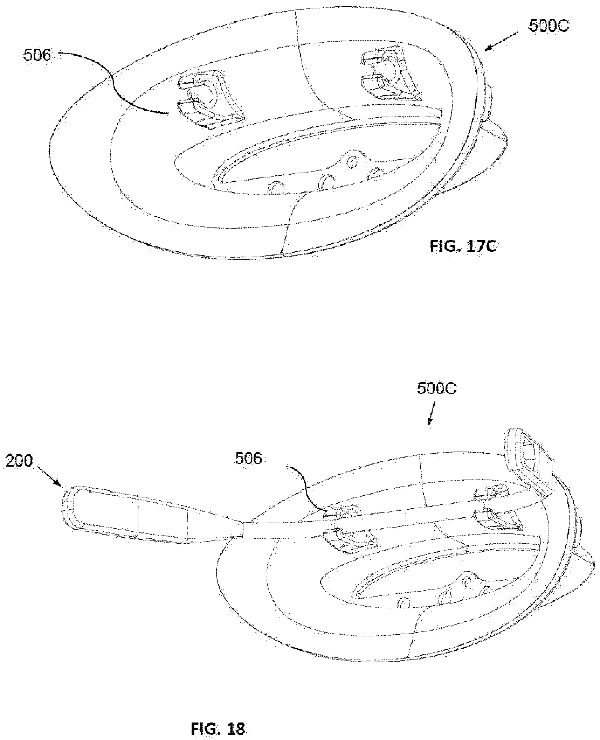

In another additional version embodiment of housing **500C** depicted in FIG. 17C, the rear or inner side of the housing has two receptor brackets with slots **506** for connecting with the front part **202** of strap **200** for fixation on to the anti-snoring device. An assembled view of this configuration is shown in FIG. 18, wherein strap **200** is attached to housing version **500C** using receptor brackets with slots **506.** It can be also used in combination with several other types of straps that are available at the front middle part generally over maxillary part of an anti-snoring or OSA ameliorating device. Typically, receptor brackets connect with the front part of a strap of an oral appliance such as an anti-snoring device or a mandibular advancement device that uses a single strap pivoted to the rear portion of mandibular part on the two ends and encircling the maxillary part at front. Those skilled in the art may appreciate that there can be several variants of these designs, the disclosed variants are just a few examples; and all those other variants are covered by the spirit and scope of this disclosure.

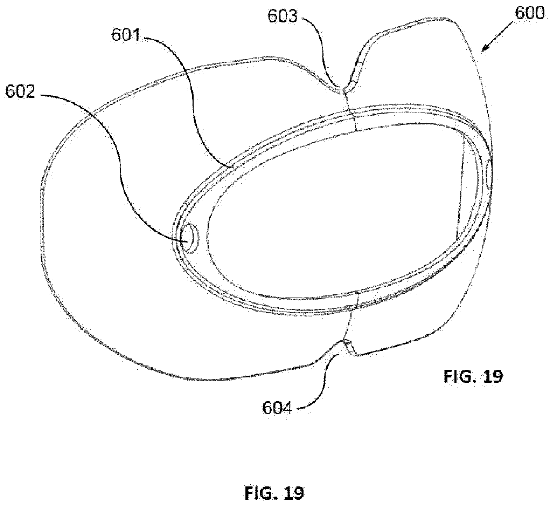

Mask component **600** (FIG. 19) is made of a soft thermoplastic, silicone rubber, nitrile rubber, or such flexible and compliant material that is configured to conform to the skin on the inside of the wearer's lips preventing air from escaping from the gaps between the lips and projecting out portion of the modular breathing assist device. The intent of this disclosed configuration is to avoid or at least minimize passage of air from other routes to enable improved control of air flow by allowing it to pass only through the modular breathing assist device. The shape is ergonomic to ensure that no foreign body sensation is felt while wearing the device. Due to its modular design, the user can choose the right size to wear, depending on the size of the mouth, and it can be attached to any anti snoring device in addition to the disclosed anti-snoring device.

The receptor cavity **601** in the mask (FIG. 19) and groove **501** in the housing (FIG. 15) are built as matching configurations for facilitating easy connection of parts **600** and **500.** Receptor cavity **602** in the mask is configured to receive protruding button **502** in the housing to secure and prevent slippage and rotation of the mask **600.**

The recess **603** on the upper side of mask and recess **604** in the lower side of the mask are for better wearing experience because there is connecting tissue present in the wearer patient's mouth at locations where the dentition connects to the lips. The recesses **603** and **604** can avoid possible discomfort caused by interference with any such tissues to enhance the wearing experience. Optionally, and in addition, the amount of recess in **603** may be deeper than that of **604** because usually the interfering tissue of the maxillary portion is relatively larger than that of the mandibular portion.

Each part of the assembly may be replaced depending on the level of wear and tear, due to the modular structure of the assembly.

It is obvious that the foregoing embodiments of the invention are examples and can be varied in many ways. Such present or future variations are not to be regarded as a departure from the spirit and scope of the invention, and all such modifications as would be obvious to one skilled in the art are intended to be included within the scope of the following claims.

## Claims

1. A system for preventing or reducing snoring or obstructive sleep apnea in a patient by controlling an amount of mandibular advancement of a mandible of the patient relative to a maxilla of the patient, the system comprising:
a mandibular part adapted for fitting engagement with one or more natural teeth, implants and/or gums on the mandible of the patient;
a maxillary part adapted for fitting engagement with one or more natural teeth, implants and/or gums on the maxilla of the patient; and
an adjustable strap assembly configured to link the mandibular part and the maxillary part to control the amount of mandibular advancement of the mandible relative to the maxilla, the adjustable strap assembly comprising:
a strap having a first end and a second end; and
a pair of casings, each said casing having an axial opening configured to receive a respective one of said first and second ends of the strap, and a lateral groove configured to receive a means for securing the strap in the axial opening of the casing at a desired position;
wherein the casings of the adjustable strap assembly are configured to connect to the mandibular part and the strap is configured to connect to the maxillary part, and
wherein the length of the strap assembly is adjustable by adjusting the position of the strap within the casing.

2. The system of claim 1, wherein the means for securing the strap comprises screws and nuts, and optionally C-shaped snap rings.

3. The system of claim 1 or 2, wherein the casings further comprise markings or gradations provided to facilitate measurement of the mandibular advancement and adjustment of the length of the strap.

4. The system of any one of claims 1 to 3, wherein said mandibular part is a lower member splint or tray structured to engage with one or more natural teeth on user's mandible, and said maxillary part is an upper member splint or tray structured to engage with one or more natural teeth on user's maxilla; or
wherein said mandibular part is a mandibular denture adapted to be worn on lower jaw of a user wherein retention of the denture on user's jaw is enhanced by use of glue between the denture and gum or by use of an implant on jaw or by use of a clamp or clasp between the denture and natural or implanted teeth, and said maxillary part is a maxillary denture adapted to be worn on upper jaw of the user wherein retention of the denture on user's jaw is enhanced by use of glue between the denture and gum or by use of an implant on jaw or by use of a clamp or clasp between the denture and natural or implanted teeth.

5. The system of any one of claims 1 to 4, wherein one or more of the mandibular part, the maxillary part, and the strap assembly are made of bio-compatible Nylon, PEEK, Co-Cr, Ti-6Al-4V alloy with or without infusion of nano-particles; or
wherein one or more of the mandibular part, the maxillary part, and the strap assembly are made by 3D printing or additive manufacturing process.

6. The system of claim 3, wherein said mandibular dentures and said maxillary dentures are removable partial dentures or removable full dentures; or
wherein said mandibular denture and said maxillary denture are implant supported dentures selected from single tooth dental implant, multiple-tooth on implant-supported bridge, and multiple-tooth on implant-retained denture.

7. The system of any one of claims 1 to 6, wherein the casings further comprises a cavity configured to attachment to a corresponding projection on the mandibular part or the maxillary part and/or wherein the maxillary part comprises a notch configured to receive a front middle part of the strap for retention by means of a snap fitting action.

8. A modular breathing assist device for preventing or reducing snoring or obstructive sleep apnea in a user, the device comprising:
a valve body comprising a plurality of openings;
a housing configured to receive the valve body in a first position and a second position, said housing having openings that partially or fully align with the plurality of openings in the valve body when the valve body is rotated between the first position and the second position;
a mask comprising a housing opening configured to receive the housing;
wherein when said valve body, mask, and housing are assembled, the device is configured for placement in the front portion of an oral cavity of the user to facilitate control of quantity of air flow from mouth during exhaling from the airway through the oral cavity of the user, and
wherein the quantity of air flow from mouth or oral cavity of the user during exhalation is controlled by the valve body.

9. The device of claim 8, wherein the quantity of air flow is adjustable by moving the valve body between the first position and the second position; or
wherein the plurality of openings on the valve body comprises a first plurality of small openings and a second plurality of large openings, wherein the quantity of air flow is adjustable by moving the valve body between the first position and the second position; or
wherein the plurality of openings on the valve body comprises a first plurality of openings and a second plurality of openings, wherein the first plurality comprises a larger number of holes than the second plurality, wherein the quantity of air flow is adjustable by moving the valve body between the first position and the second position.

10. The device of any one of claims 8 or 9, wherein the valve body is connected to the housing via a snap fit connection.

11. The device of any one of claims 8 to 10, wherein the mask further comprises a mating cavity on an inner periphery of the housing opening configured to mate with a corresponding groove on an outer periphery of the housing to secure the housing to the mask.

12. The device of any one of claims 8 to 11, wherein the housing further comprises at least one protruding button configured for insertion into a corresponding receptor cavity on the mask to secure the housing to the mask.

13. The device of any one of claims 8 to 12, wherein the housing on a rear surface has at least one C-shaped clasp configured to latch on to the strap of the mandibular advancement system as defined in any one of claims 1 to 7; or
wherein the housing on a rear surface has at least one C-shaped clasp configured to latch on to the strap of the mandibular advancement system as defined in any one of claims 1 to 7 and wherein the housing and mask are configured to retain the mandibular advancement system at the front of the oral cavity of the user between lips and dentition of the user.

14. The device of any one of claims 8 to 13, wherein one or more of the valve body, the mask, the housing are made of bio-compatible Nylon, PEEK, or rubber and made using 3D-printing, additive manufacturing, or injection molding.

15. A method of using a system as defined in any one of claims 1 to 7 to prevent or reduce snoring or obstructive sleep apnea in a patient, the method comprising the steps of:
engaging the mandibular part with one or more natural teeth, implants and/or gums on the mandible of the patient;
engaging the maxillary part with one or more natural teeth, implants and/or gums on the maxilla of the patient;
connecting the mandibular part and the maxillary part using the adjustable strap assembly by attaching each said casing to a rear portion of the mandibular part and snapping the strap into a notch located at a front portion of the maxillary part;
adjusting the effective strap length by moving the strap within the casing to achieve adequate mandibular advancement to prevent or reduce snoring or obstructive sleep apnea while considering tolerance and comfort of the patient; and
when desired strap length is achieved, tightening the screw to lock the strap within the casing.
